(19) Europäisches Patentamt
European Patent Office
Office européen des brevets

(11) Veröffentlichungsnummer: **0 064 658**
**A1**

(12) # EUROPÄISCHE PATENTANMELDUNG

(21) Anmeldenummer: **82103514.4**

(22) Anmeldetag: **26.04.82**

(51) Int. Cl.³: **C 07 C 79/35**
C 07 D 317/22, C 07 C 76/02
A 01 N 35/04, A 01 N 43/24
//C07D319/06, C07D321/06

(30) Priorität: **09.05.81 DE 3118371**

(43) Veröffentlichungstag der Anmeldung:
**17.11.82 Patentblatt 82/46**

(84) Benannte Vertragsstaaten:
**AT BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Förster, Heinz, Dr.**
**Am Eckbusch 47**
**D-5600 Wuppertal 1(DE)**

(72) Erfinder: **Eue, Ludwig, Dr.**
**Paul-Klee-Strasse 36**
**D-5090 Leverkusen 1(DE)**

(72) Erfinder: **Schmidt, Robert, Dr.**
**Im Waldwinkel 110**
**D-5060 Bergisch Gladbach 2(DE)**

(72) Erfinder: **Lürssen, Klaus, Dr.**
**August-Kierspel-Strasse 89**
**D-5060 Bergisch Gladbach 2(DE)**

(54) **Substituierte Phenoxybenzaldehyd-acetale, Verfahren zu deren Herstellung und deren Verwendung als Herbizide und Pflanzenwuchsregulatoren.**

(57) Substituierte Phenoxybenzaldehyd-acetale der Formel

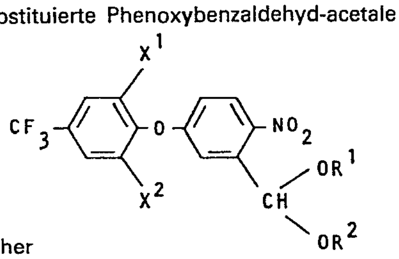

(1)

in welcher
$X^1$ für Wasserstoff oder Halogen steht,
$X^2$ für Wasserstoff oder Halogen steht,
$R^1$ für gegebenenfalls substituiertes Alkyl steht und
$R^2$ für gegebenenfalls substituiertes Alkyl steht und außerdem
$R^1$ und $R^2$ gemeinsam für gegebenenfalls substituiertes Alkylen stehen,
besitzen sehr gute herebizide und pflanzenwuchsregulierende Eigenschaften. Die erfindungsgemäßen Stoffe lassen sich nach mehreren Verfahren herstellen.

EP 0 064 658 A1

Croydon Printing Company Ltd.

BAYER AKTIENGESELLSCHAFT 5090 Leverkusen, Bayerwerk

Zentralbereich
Patente, Marken und Lizenzen Dü/ABc
Ib

Substituierte Phenoxybenzaldehyd-acetale, Verfahren zu deren Herstellung und deren Verwendung als Herbizide und Pflanzenwuchsregulatoren

Die Erfindung betrifft neue substituierte Phenoxybenzaldehyd-acetale, mehrere Verfahren zu deren Herstellung sowie deren Verwendung als Herbizide und Pflanzenwuchsregulatoren.

Es ist bekannt, daß zahlreiche substituierte Diphenylether herbizide Eigenschaften besitzen. So eignet sich zum Beispiel $\mathcal{L}$-(5-(2-Chlor-4-trifluormethyl-phenoxy)-2-nitro-phenoxy)-propionsäureethylester zur Bekämpfung von Unkraut (vgl. DE-OS 2 311 638 und US-PS 4 093 446). Die Wirkung dieses Stoffes gegen Unkräuter ist gut, jedoch ist die selektive herbizide Wirksamkeit nicht immer ausreichend.

Es wurden nun neue substituierte Phenoxybenzaldehyd-acetale der Formel

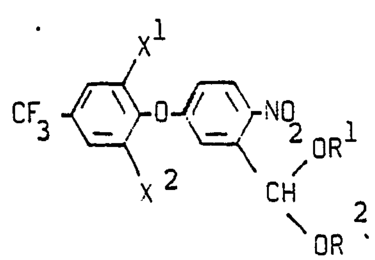

(I)

in welcher

$X^1$ für Wasserstoff oder Halogen steht,

$X^2$ für Wasserstoff oder Halogen steht,

$R^1$ für gegebenenfalls substituiertes Alkyl steht und

$R^2$ für gegebenenfalls substituiertes Alkyl steht und außerdem

$R^1$ und $R^2$ gemeinsam für gegebenenfalls substituiertes Alkylen steht,

gefunden.

Weiter wurde gefunden, daß man die neuen substituierten Phenoxybenzaldehyd-acetale der Formel (I) enthält, wenn man

(a) Phenoxybenzaldehyde der Formel

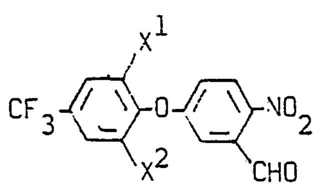

(II)

in welcher

$X^1$ und $X^2$ die oben angegebene Bedeutung haben,

entweder

α) mit Alkoholen der Formel

$$R^1OH \qquad (IIIa)$$

in welcher
$R^1$ die oben angegebene Bedeutung hat,

und/oder der Formel

$$R^2OH \qquad (IIIb)$$

in welcher
$R^2$ die oben angegebene Bedeutung hat,

bzw. mit Alkandiolen der Formel

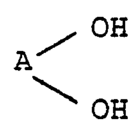

(IV)

in welcher
A für gegebenenfalls substituiertes Alkylen steht,

gegebenenfalls in Gegenwart eines Katalysators und gegebenenfalls in Gegenwart eines Verdünnungsmittels umsetzt,

oder

ß) mit Orthoameisensäureestern der Formel

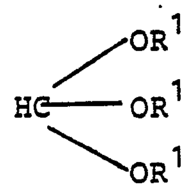

(V)

in welcher
$R^1$ die oben angegebene Bedeutung hat,

in Gegenwart eines Katalysators und gegebenenfalls in Gegenwart eines Verdünnungsmittels umsetzt,

oder

b) Phenoxybenzaldehyd-acetale der Formel

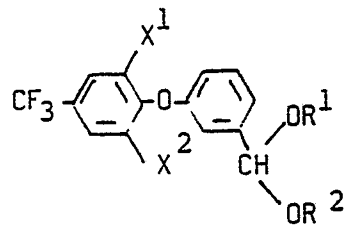

(VI)

in welcher
$X^1$, $X^2$, $R^1$ und $R^2$ die oben angegebene Bedeutung haben,

mit Salpetersäure gegebenenfalls in Gegenwart eines Katalysators und gegebenenfalls in Gegenwart eines Verdünnungsmittels umsetzt,
oder

(c) Phenoxybenzaldehyd-acylale der Formel

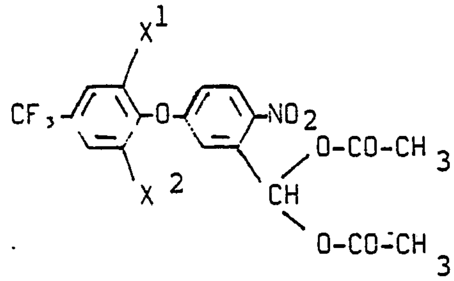

(VII)

in welcher
$X^1$ und $X^2$ die oben angegebene Bedeutung haben,

mit Alkoholen der Formel

$$R^1OH \qquad (IIIa)$$

in welcher
$R^1$ die oben angegebene Bedeutung hat,

und/oder der Formel

$$R^2OH \qquad (IIIb)$$

in welcher
$R^2$ die oben angegebene Bedeutung hat,

bzw. mit Alkandiolen der Formel

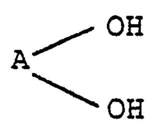
(IV)

in welcher
A die oben angegebene Bedeutung hat,

gegebenenfalls in Gegenwart eines Katalysators und gegebenenfalls in Gegenwart eines Verdünnungsmittels umsetzt, oder

(d) Phenole der Formel

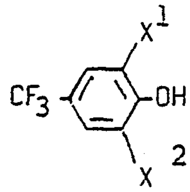

(VIII)

in welcher

$X^1$ und $X^2$ die oben angegebene Bedeutung haben,

mit 5-Halogen-2-nitro-benzaldehyd-acetalen der Formel

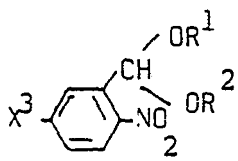

(IX)

in welcher

$R^1$ und $R^2$ die oben angegebene Bedeutung haben und

$X^3$ für Fluor, Chlor oder Brom steht,

gegebenenfalls in Gegenwart eines Säureakzeptors und gegebenenfalls in Gegenwart eines Verdünnungsmittels umsetzt.

Schließlich wurde gefunden, daß sich die neuen substituierten Phenoxybenzaldehyd-acetale der Formel (I) durch sehr gute herbizide und pflanzenwuchsregulierende Wirksamkeit auszeichnen. Sie können insbesondere zur selektiven Unkrautbekämpfung in wichtigen Kulturen, wie zum Beispiel in Mais, Sojabohnen, Getreide und Baumwolle eingesetzt werden. Die pflanzenwuchsregulierende Wirkung der neuen Stoffe kann vor allem in der Anwendung als Defoliants und Desiccants bei Baumwolle genutzt werden.

Überraschenderweise besitzen die erfindungsgemäßen substituierten Phenoxybenzaldehydacetale der Formel (I) eine wesentlich bessere herbizide und pflanzenwuchsregulierende Wirksamkeit als aus dem Stand der Technik bekannte Verbindungen analoger Konstitution und gleicher Wirkungsrichtung. So übertreffen die erfindungsgemäßen Stoffe den bekannten $\mathcal{L}$-(5-(2-Chlor-4-trifluormethyl-phenoxy)-2-nitro-phenoxy)-propionsäure-ethylester bezüglich seiner selektiven herbiziden Eigenschaften.

Die erfindungsgemäßen substituierten Phenoxybenzaldehydacetale sind durch Formel (I) definiert. In dieser Formel stehen vorzugsweise

$X^1$ für Chlor,

$X^2$ für Wasserstoff oder Chlor,

$R^1$ für gegebenenfalls ein- oder mehrfach durch Fluor, Chlor, Methoxy und/oder Ethoxy substituiertes, geradkettiges oder verzweigtes Alkyl mit 1 bis 5 Kohlenstoffatomen und

$R^2$ für gegebenenfalls ein- oder mehrfach durch Fluor, Chlor, Methoxy und/oder Ethoxy substituiertes geradkettiges oder verzweigtes Alkyl mit 1 bis 5 Kohlenstoffatomen und außerdem

$R^1$ und $R^2$ gemeinsam für gegebenenfalls durch Alkyl mit 1 bis 3 Kohlenstoffatomen, Chloralkyl mit 1 oder 2 Kohlenstoffatomen und 1 bis 3 Chloratomen und/oder Chlor substituiertes Alkylen mit 2 bis 5 Kohlenstoffatomen.

Verwendet man bei dem erfindungsgemäßen Verfahren (a) 5-(2,6-Dichlor-4-trifluormethyl-phenoxy)-2-nitro-benzaldehyd und Propandiol (Variante α) bzw. Orthoameisensäure-triethylester (Variante ß) als Ausgangsstoffe, bei dem erfindungsgemäßen Verfahren (b) 3-(2-Chlor-4-trifluormethyl-phenoxy)-benzaldehyd-diisobutylacetal und Salpetersäure als Ausgangsstoffe, bei dem erfindungsgemäßen Verfahren (c) 5-(2,6-Dichlor-4-trifluormethyl-phenoxy)-2-nitrobenzaldehyd- diacetacylal und Methanol als Ausgangsstoffe und bei dem erfindungsgemäßen Verfahren (d) 2,6-Dichlor-4-trifluor-methyl-phenol und 5-Fluor-2-nitro-benzaldehyd-dimethylacetal als Ausgangsstoffe, so kann der Verlauf der erfindungsgemäßen Verfahren durch die folgenden Formelschemate wiedergegeben werden:

(a), Variante α :

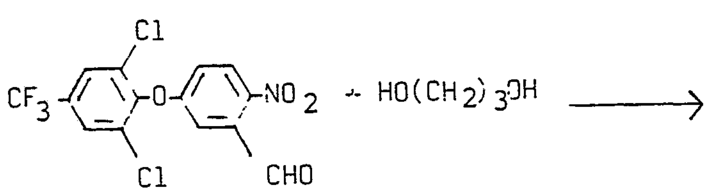

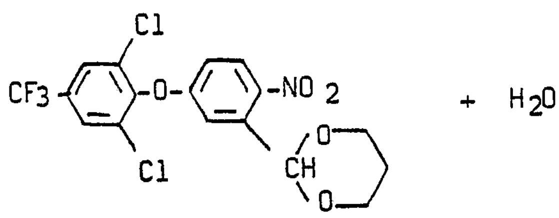

(a), Variante ß:

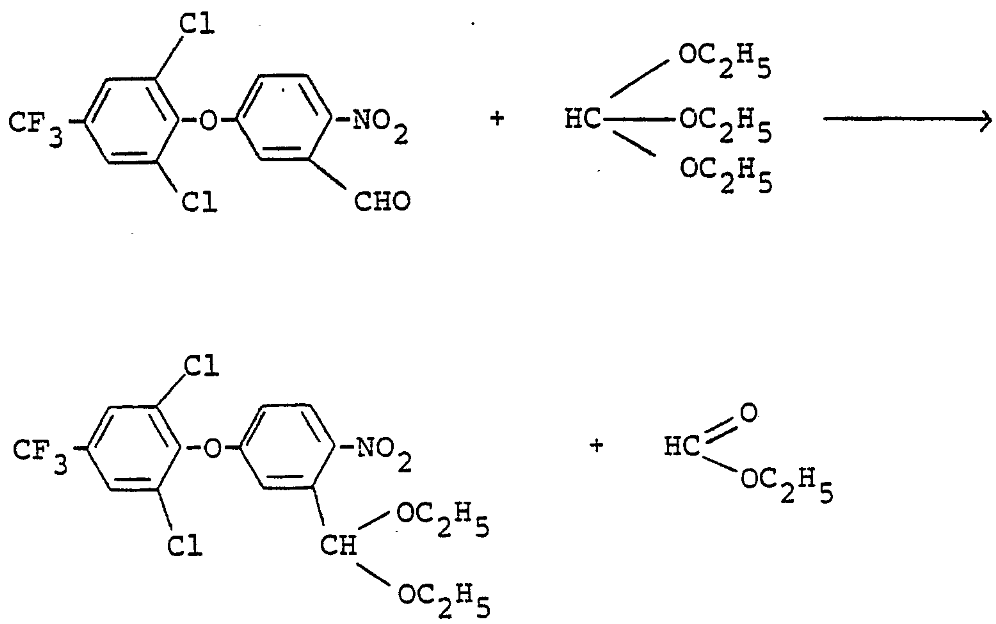

(b)

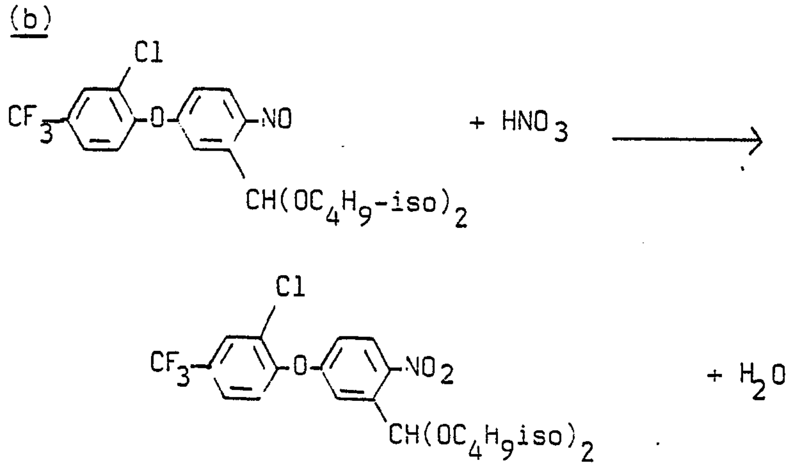

(c)

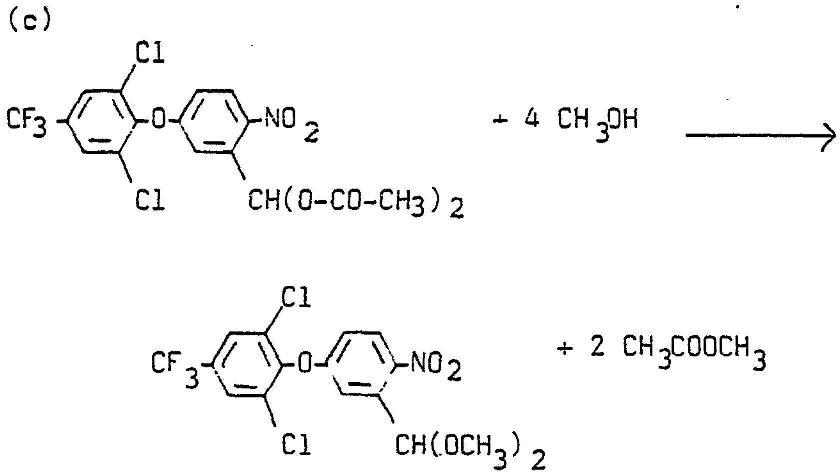


(d)

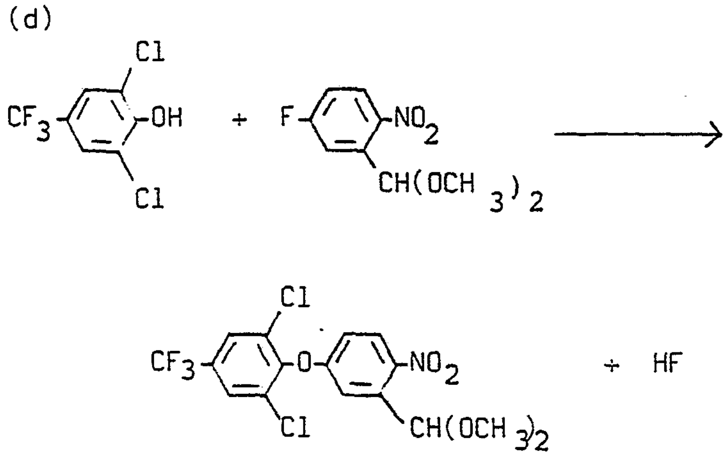


Die bei dem erfindungsgemäßen Verfahren (a) als Ausgangsstoffe zu verwendenden Phenoxybenzaldehyde sind durch die Formel (II) definiert. In dieser Formel haben $X^1$ und $X^2$ vorzugsweise diejenigen Bedeutungen, die bereits im Zusammenhang mit der Beschreibung der erfindungsgemäßen Phenoxybenzaldehyd-acetale der Formel (I) vorzugsweise für diese Reste genannt wurden.

Als Beispiele für Phenoxybenzaldehyde der Formel (II) seien 5-(2-Chlor-4-trifluormethyl-phenoxy)-2-nitro-

benzaldehyd und 5-(2,6-Dichlor-4-trifluormethylphenoxy)-2-nitro-benzaldehyd genannt.

Die Verbindungen der Formel (II) sind bereits bekannt (vgl. DE-OS 3 017 795).

Die bei dem erfindungsgemäßen Verfahren (a), Variante α, als Reaktionskomponenten benötigten Alkohole beziehungsweise Alkandiole sind durch die Formeln (IIIa), (IIIb) und (IV) allgemein definiert. In den Formeln (IIIa) und (IIIb) haben $R^1$ und $R^2$ vorzugsweise diejenigen Bedeutungen, die bereits im Zusammenhang mit der Beschreibung der erfindungsgemäßen Stoffe der Formel (I) vorzugsweise für diese Reste einzeln angegeben wurden. A steht in der Formel (IV) vorzugsweise für gegebenenfalls durch Alkyl mit 1 bis 3 Kohlenstoffatomen, Chloralkyl mit 1 oder 2 Kohlenstoffatomen und 1 bis 3 Chloratomen und/oder Chlor substituiertes Alkylen mit 2 bis 5 Kohlenstoffatomen.

Als Beispiele für Alkohole der Formeln (IIIa) und (IIIb) sowie für Alkandiole der Formel (IV) seien genannt:

Methanol, Ethanol, n- und iso-Propanol, n-, iso- und sec-Butanol, n-, iso- und sec-Pentanol, 2,2,2-Trifluor-ethanol, 2-Methoxyethanol, 2-Ethoxy-ethanol, 2-Chlor-ethanol, 2,2,2-Trichlorethanol, Ethan-1,2-diol, Propan-1,2-diol, Propan-1,3-diol, Butan-1,2-diol, Butan-1,3-diol, Butan-2,3-diol, 2-Methyl-propan-1,3-diol, 2,2-Dimethylpropan-1,3-diol, Butan-1,4-diol, 2-Methyl-butan-1,4-diol, 2-Chlor-butan-1,4-diol, 1-Methyl-butan-1,4-diol, 1-Chlor-butan-1,4-diol, 1,4-Dimethyl-butan-1,4-diol und 2,3-Dimethyl-butan-2,3-diol.

Die Verbindungen der Formeln (IIIa), (IIIb) und (IV) sind bekannt.

Die bei dem erfindungsgemäßen Verfahren (a), Variante ß, als Reaktionskomponenten benötigten Orthoameisensäureester sind durch die Formel (V) allgemein definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden.

Als Beispiele für Orthoameisensäureester der Formel (V) seien im einzelnen genannt:

Orthoameisensäure-trimethylester
Orthoameisensäure-triethylester
Orthoameisensäure-tri-n-propylester.

Die Orthoameisensäureester der Formel (V) sind bekannt oder lassen sich nach im Prinzip bekannten Methoden in einfacher Weise herstellen.

Die bei dem Verfahren (b) als Ausgangstoffe zu verwendenden Phenoxy-benzaldehyd-acetale sind durch Formel (VI) definiert. In dieser Formel haben $X^1$, $X^2$, $R^1$ und $R^2$ vorzugsweise für diejenigen Bedeutungen, welche im Rahmen der Beschreibung der erfindungsgemäßen Verbindungen der Formel (I) als bevorzugt angegeben sind für diese Reste.

Als Beispiele für Phenoxybenzaldehyd-acetale der Formel (VI) seien im einzelnen genannt:

3-(2-Chlor-4-trifluormethyl-phenoxy)-benzaldehyd- und 3-(2,6-Dichlor-4-trifluormethyl-phenoxy)-benzaldehyd-dimethylacetal, -diethylacetal, -di-n-propylacetal, -ethylenglycolacetal und -propylenglycolacetal.

Die Verbindungen der Formel (VI) sind bereits bekannt (vgl. DE-OS 3 017 795).

Die bei dem Verfahren (c) als Ausgangsstoffe zu verwendenden Phenoxy-benzaldehyd-acylale sind durch die Formel (VII) definiert. In dieser Formel stehen vorzugsweise

$X^1$ für Chlor und
$X^2$ für Wasserstoff oder Chlor.

Als Beispiele seien 5-(2-Chlor-4-trifluormethyl-phenoxy)-2-nitro-benzaldehyd-diacetacylal und 5-(2,6-Dichlor-4-trifluormethyl-phenoxy)-2-nitro-benzaldehyd-diacetacylal genannt.

Die Phenoxybenzaldehyd-acylale der Formel (VII) sind Gegenstand einer vorgängigen, noch nicht veröffentlichten Patentanmeldung. Man erhält diese Verbindungen, wenn man Phenoxybenzaldehyde der Formel

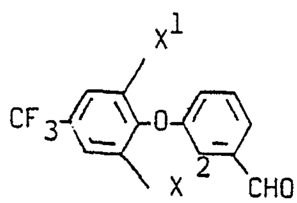 (X)

in welcher
$X^1$ und $X^2$ die oben angegebene Bedeutung haben,

mit Acetanhydrid, gegebenenfalls in Gegenwart eines Verdünnungsmittels, wie z.B. Methylenchlorid, bei Temperaturen zwischen lo und

80°C zunächst zu den entsprechenden Acylalen der Formel

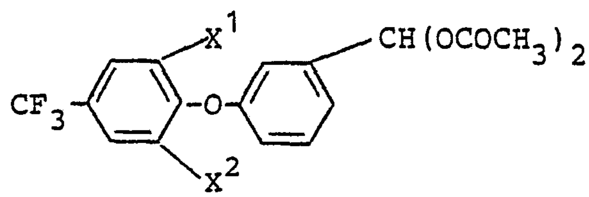

(XI)

in welcher
$X^1$ und $X^2$ die oben angegebene Bedeutung haben,

umsetzt, und diese dann nitriert, indem man bei Temperaturen zwischen -10 und +20°C Salpetersäure dazu gibt, nach Ende der Umsetzung mit Wasser verdünnt, gegebenenfalls das nicht-wäßrige Verdünnungsmittel abtrennt oder abdestilliert und die kristallin anfallenden Produkte der Formel (VII) durch Filtration isoliert.

Die Verbindungen der Formel (XI) lassen sich auch aus den Acetalen der Formel (VI) durch Umsetzung mit 4 Äquivalenten Essigsäure pro Mol Acetal der Formel (VI) herstellen (vgl. Houben-Weyl, Bd. VII/1, Seite 443).

Als Beispiele für die Phenoxybenzaldehyde der Formel (X) seien genannt:

3-(2-Chlor-4-trifluormethyl-phenoxy)-benzaldehyd und 3-(2,6-Dichlor-4-trifluormethyl-phenoxy)-benzaldehyd.

Die Phenoxybenzaldehyde der Formel (X) erhält man, wenn man 3-Hydroxy-benzaldehyd mit halogen-substituiertem Benzotrifluorid der Formel

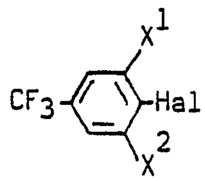

(XII)

in welcher

$X^1$ und $X^2$ die oben angegebene Bedeutung haben und

Hal für Chlor steht ,

in Gegenwart eines Säurebindemittels, wie z.B. Kaliummmethylat und in Gegenwart von Verdünnungsmitteln, wie z.B. Dimethylsulfoxid und Toluol, bei Temperaturen zwischen 5o und 12o°C umsetzt. Die Aufarbeitung kann nach üblichen Methoden durchgeführt werden, beispielsweise durch Abdestillieren der flüchtigen Komponenten, Digerieren des Rückstandes mit Toluol, Filtration, Waschen und Einengen des Filtrats. Der hierbei erhaltene Rückstand enthält im wesentlichen die Produkte der Formel (X), welche über ihre Bisulfit-Addukte gereinigt werden können.

Die bei der Durchführung des erfindungsgemäßen Verfahrens (c) als Reaktionskomponenten benötigten Alkohole der Formeln (IIIa) und (IIIb) beziehungsweise die Alkandiole der Formel (IV) wurden bereits im Zusammenhang mit der Beschreibung des erfindungsgemäßen Verfahrens (a), Variante α , im einzelnen abgehandelt.

Die bei dem erfindungsgemäßen Verfahren (d ) als Ausgangsstoffe benötigten Phenole sind durch die Formel (VIII) definiert. In dieser Formel haben $X^1$ und $X^2$ vorzugsweise diejenigen Bedeutungen, die bereits im Zusammenhang mit der Beschreibung der erfindungsgemäßen Stoffe der Formel

(I) vorzugsweise für diese Reste genannt wurden. Die Phenole der Formel (VIII) sind bekannt (vgl. EP-OS 19388).

Die bei dem erfindungsgemäßen Verfahren (d) als Reaktionskomponenten benötigten 5-Halogen-2-nitro-benzaldehyd-acetale sind durch die Formel (IX) definiert. In dieser Formel haben $R^1$ und $R^2$ vorzugsweise diejenigen Bedeutungen, die bereits im Zusammenhang mit der Beschreibung der erfindungsgemäßen Stoffe der Formel (I) vorzugsweise für diese Reste genannt wurden. $X^3$ steht vorzugsweise für Chlor oder Brom.

Die 5-Halogen-2-nitro-benzaldehyd-acetale der Formel (IX) sind bekannt oder lassen sich in einfacher Weise nach im Prinzip bekannten Verfahren herstellen. So erhält man die Verbindungen der Formel (IX) beispielsweise dadurch, daß man 3-Halogen-benzaldehyd in üblicher Weise zunächst acetalisiert und dann nitriert.

Das erfindungsgemäße Verfahren (a) wird sowohl beim Arbeiten nach der Variante $\alpha$ als auch nach der Variante ß vorzugsweise unter Verwendung eines Katalysators durchgeführt. Als Reaktionsbeschleuniger können hierbei alle üblichen, für Acetalisierungen geeigneten Katalysatoren eingesetzt werden. Vorzugsweise in Frage kommen Schwefelsäure, p-Toluolsulfonsäure, Chlorwasserstoff und Ammoniumchlorid (vgl. Methodicum Chimicum, Band 5, Seiten 513-516, Georg Thieme Verlag, Stuttgart/Academic Press New York, San Franzisco, London, 1975).

Ferner wird das erfindungsgemäße Verfahren (a) sowohl

beim Arbeiten nach der Variante α als auch nach der Variante ß vorzugsweise unter Verwendung von Verdünnungsmitteln durchgeführt, welche zur Entfernung von Wasser durch azeotrope Destillation geeignet sind. Als Beispiele hierfür seien Benzol, Toluol und Xylol genannt. Toluol wird besonders bevorzugt.

Bei der Durchführung des erfindungsgemäßen Verfahrens (a) können die Reaktionstemperaturen sowohl beim Arbeiten nach der Variante α als auch nach der Variante ß innerhalb eines größeren Bereiches variiert werden. Im allgemeinen arbeitet man bei Temperaturen zwischen 0°C und 200°C, vorzugsweise zwischen 20°C und 150°C.

Das Verfahren (a) wird sowohl nach Variante α als auch nach Variante ß im allgemeinen unter Normaldruck durchgeführt.

Bei der Durchführung des erfindungsgemäßen Verfahrens (a) nach der Variante α setzt man pro Mol an Phenoxybenzaldehyd der Formel (II) 2 bis 10 Mol, vorzugsweise 3 bis 5 Mol Alkohol der Formel (IIIa) und/oder (IIIb) beziehungsweise 1 bis 5 Mol, vorzugsweise 1,5 bis 3 Mol an Alkandiol der Formel (IV) ein. Im einzelnen arbeitet man vorzugsweise so, daß man Phenoxybenzaldehyd der Formel (II) mit Alkoholen der Formel (IIIa) und/oder (IIIb) beziehungsweise mit Alkandiol der Formel (IV), mit einem Katalysator und einem Verdünnungsmittel versetzt, das Reaktionsgemisch bis zum Ende der Umsetzung zum Sieden erhitzt und gegebenenfalls das Reaktionswasser am Wasserabscheider entfernt. Zur Aufarbeitung wird

gegebenenfalls eingeengt, mit Toluol verdünnt, gegebenenfalls mit Wasser gewaschen, getrocknet, filtriert und eingeengt. Das als Rückstand verbleibende Produkt der Formel (I) wird durch den Schmelzpunkt oder den Brechungsindex charakterisiert.

Bei der Durchführung des erfindungsgemäßen Verfahrens (a) nach der Variante ß setzt man pro Mol an Phenoxybenzaldehyd der Formel (II) 2 bis 10 Mol, vorzugsweise 3 bis 5 Mol an Orthoameisensäureester der Formel (V) ein. Im einzelnen arbeitet man so wie es bereits im Zusammenhang mit der Beschreibung der Variante $\beta$ geschildert wurde.

Das erfindungsgemäße Verfahren (b) wird vorzugsweise unter Verwendung eines Katalysators durchgeführt. Insbesondere kommt als Katalysator hierbei Schwefelsäure in Frage.

Als Verdünnungsmittel kommen bei dem erfindungsgemäßen Verfahren (b) alle unter den Reaktionsbedingungen inerten Solventien in Betracht. Vorzugsweise verwendbar ist Methylenchlorid.

Die Reaktionstemperaturen können bei der Durchführung des erfindungsgemäßen Verfahrens (b) in einem bestimmten Bereich variiert werden. Im allgemeinen arbeitet man bei Temperaturen zwischen -20°C und +50°C, vorzugsweise zwischen -10°C und +30°C.

Bei der Durchführung des erfindungsgemäßen Verfahrens (b) versetzt man Phenoxybenzaldehyd-acetal der Formel (VI) gegebenenfalls in Gegenwart eines Katalysators und gegebenenfalls in Gegenwart eines Verdünnungsmittels mit Salpetersäure, und zwar so daß pro Mol an Phenoxybenzaldehyd-acetal der Formel (VI) 1 bis 3 Mol, vorzugsweise 1,2 bis 1,5 Mol an Salpetersäure enthalten sind. Die Aufarbeitung erfolgt nach üblichen Methoden. Im allgemeinen verfährt man in der Weise, daß man das Reaktionsgemisch mit einem mit Wasser praktisch nicht mischbaren Lösungsmittel, wie zum Beispiel Methylenchlorid, verdünnt, die organische Phase abtrennt, mit Wasser wäscht, trocknet, filtriert und einengt. Die Stoffe der Formel (I) verbleiben dabei als Rückstand.

Das erfindungsgemäße Verfahren (c) wird vorzugsweise in Gegenwart eines Katalysators und in Gegenwart eines Verdünnungsmittels durchgeführt. Vorzugsweise in Frage kommen hierbei diejenigen Katalysatoren und Verdünnungsmittel, die bereits im Zusammenhang mit der Beschreibung des erfindungsgemäßen Verfahrens (a) vorzugsweise als Katalysatoren bzw. Verdünnungsmittel genannt wurden.

Bei der Durchführung des Verfahrens (c) können die Reaktionstemperaturen innerhalb eines größeren Bereiches variiert werden, und zwar so wie es bereits im Zusammenhang mit dem erfindungsgmeäßen Verfahren (a) angegeben wurde.

Bei der Durchführung des erfindungsgemäßen Verfahrens (c) setzt man auf 1 Mol Phenoxybenzaldehyd-acylal der

Formel (VII) mindestens 4 Moläquivalente der Alkohole der Formeln (IIIa) und/oder (IIIb) bzw. an Alkandiol der Formel (IV) ein. Vorzugsweise setzt man jedoch einen größeren Überschuß an der alkoholischen Komponente ein. Im einzelnen verfährt man bei der Durchführung des erfindungsgemäßen Verfahrens (c) in der Weise, daß man die Komponenten vermischt, zum Sieden erhitzt und die als Koppelprodukte gebildeten Ester sowie gegebenenfalls überschüssige Alkohole und Solventien abdestilliert, wobei die Verbindungen der Formel (I) im Rückstand verbleiben.

Als Säureakzeptoren kommen bei dem erfindungsgemäßen Verfahren (d) alle üblichen Säurebindemittel in Betracht. Vorzugsweise verwendbar sind Alkalimetall- und Erdalkalimetall-hydroxide bzw. -oxide, wie Natriumhydroxid, Kaliumhydroxid und Calciumoxid, und außerdem Alkalimetallcarbonat, wie Natriumcarbonat und Kaliumcarbonat.

Als Verdünnungsmittel kommen bei dem erfindungsgemäßen Verfahren (d) inerte organische Solventien in Betracht. Vorzugsweise verwendbar sind polare Lösungsmittel, wie Dimethylformamid und Acetonitril.

Die Reaktionstemperaturen können auch bei dem erfindungsgemäßen Verfahren (d) innerhalb eines größeren Bereiches variiert werden. Im allgemeinen arbeitet man bei Temperaturen zwischen 50°C und 150°C, vorzugsweise zwischen 60°C und 120°C.

Bei der Durchführung des erfindungsgemäßen Verfahrens

(d) setzt man auf 1 Mol Phenol der Formel (VIII) 1 Mol oder auch einen Überschuß an 5-Halogen-2-nitro-benzaldehyd-acetal der Formel (IX) ein. Die Aufarbeitung des Reaktionsgemisches erfolgt nach üblichen Methoden.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitestenSinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkraüter der Gattungen: Echinochloa, Setaria, Panicum, Digitaria, Phleum, Poa, Festuca, Eleusine, Brachiaria, Lolium, Bromus, Avena, Cyperus, Sorghum, Agrophyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe besitzen außerdem eine sehr gute pflanzenwuchsregulierende Wirksamkeit. Sie eignen sich besonders gut zur Entlaubung und Austrocknung der Blätter bei Baumwolle.

Die Wirkstoffe können in die üblichen Formulierungen übergeführt werden, wie Lösungen, Emulsionen,Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischungen mit bekannten herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können bei einer Anwendung als Herbizide sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Für die Anwendung der erfindungsgemäßen Wirktoffe als Pflanzenwuchsregulatoren gilt, daß die Anwendung in einem bevorzugten Zeitraum vorgenommen wird, dessen genaue Abgrenzung sich nach den klimatischen und vegetativen Gegebenenheiten richtet. In jedem Fall erfolgt die Anwendung als Wachstumsregulatoren im Nachauflauf-Verfahren.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Wirkstoffe geht aus den folgenden Beispielen hervor.

Herstellungsbeispiele

Beispiel 1

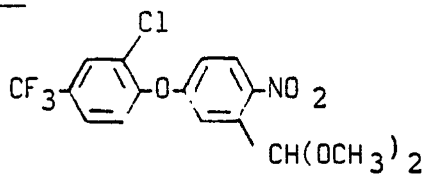

15 ml Orthoameisensäuretrimethylester werden bei 2o bis 25°C zu einer Mischung aus 14 g (0,04 Mol) 5-(2-Chlor-4-trifluormethyl-phenoxy)-2-nitro-benzaldehyd, 0,3 g Ammoniumchlorid und 5o ml Toluol gegeben. Das Reaktionsgemisch wird 16 Stunden unter Rückfluß zum Sieden erhitzt, dann filtriert und eingeengt. Der Rückstand wird in 15o ml Toluol gelöst, mit Aktivkohle geklärt, filtriert und eingeengt. Man erhält 14,8 g (94,5% der Theorie) 5-(2-Chlor-4-trifluormethyl-phenoxy)-2-nitro-benzaldehyd-dimethylacetal vom Brechungsindex $n_D^{2o}$: 1,5426.

Beispiel 2

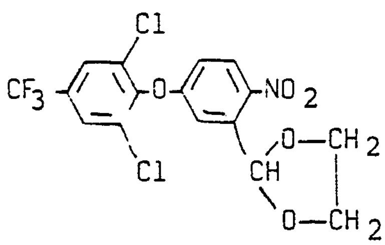

Eine Mischung aus 28,5g (0,075 Mol) 5-(2,6-Dichlor-4-trifluormethyl-phenoxy)-2-nitro-benzaldehyd, 9,3 g (0,15 Mol) Ethan-1,2-diol, 3 Tropfen konz. Schwefelsäure und 200 ml Toluol wird 12 Stunden unter Rückfluß am Wasserabscheider zum Sieden erhitzt. Das Reaktionsgemisch wird nach Erkalten mit Wasser gewaschen, getrocknet, filtriert und eingeengt. Man erhält 22, 0 g (69% der Theorie) 5-(2,6-Dichlor-4-trifluormethyl-phenoxy)-2-nitro-benzaldehyd-ethylenglycolacetal vom Schmelzpunkt 81°C.

Analog Beispiel 1 oder 2 werden die in der nachstehenden Tabelle 1 aufgeführten Verbindungen der Formel (I) hergestellt :

Tabelle 1

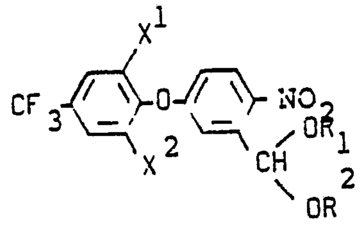

(I)

| Bei-spiel Nr. | $X^1$ | $X^2$ | $R^1$ | $R^2$ | Schmelzpunkt (°C) bzw.Brechungs-index ($n_D^{20}$) |
|---|---|---|---|---|---|
| 3 | Cl | H | $-CH_2-CH_2-$ | | 1,5569 |
| 4 | Cl | H | $-C_2H_5$ | $-C_2H_5$ | |
| 5 | Cl | Cl | $-C_2H_5$ | $-C_2H_5$ | |
| 6 | Cl | H | $-C_3H_7$-n | $-C_3H_7$-n | |
| 7 | Cl | Cl | $-C_3H_7$-n | $-C_3H_7$-n | |
| 8 | Cl | H | $-C_3H_7$-iso | $-C_3H_7$-iso | |
| 9 | Cl | Cl | $-C_3H_7$-iso | $-C_3H_7$-iso | |
| 1o | Cl | H | $-CH_2-CF_3$ | $-CH_2CF_3$ | |
| 11 | Cl | Cl | $-CH_2CF_3$ | $-CH_2CF_3$ | |

Tabelle 1 (Fortsetzung)

| Beispiel Nr. | $X^1$ | $X^2$ | $R^1$ | $R^2$ | Schmelzpunkt (°C) bzw. Brechungsindex ($n_D^{20}$) |
|---|---|---|---|---|---|
| 12 | Cl | H | $-CH_2-CH_2-OCH_3$ | $-CH_2-CH_2-OCH_3$ | |
| 13 | Cl | Cl | $-CH_2-CH_2-OCH_3$ | $-CH_2-CH_2-OCH_3$ | |
| 14 | Cl | H | $-C_4H_9-n$ | $-C_4H_9-n$ | |
| 15 | Cl | Cl | $-C_4H_9-n$ | $-C_4H_9-n$ | |
| 16 | Cl | H | $-C_4H_9-iso$ | $-C_4H_9-iso$ | |
| 17 | Cl | Cl | $-C_4H_9-iso$ | $-C_4H_9-iso$ | |
| 18 | Cl | H | $-C_4H_9-sec$ | $-C_4H_9-sec$ | |
| 19 | Cl | Cl | $-C_4H_9-sec$ | $-C_4H_9-sec$ | |
| 2o | Cl | H | $-\underset{CH_3}{CH}-CH_2-$ | | |
| 21 | Cl | Cl | $-\underset{CH_3}{CH}-CH_2-$ | | |
| 22 | Cl | H | $-\underset{C_2H_5}{CH}-CH_2-$ | | |

Tabelle 1 (Fortsetzung)

| Beispiel Nr. | $X^1$ | $X^2$ | $R^1$ | $R^2$ | Schmelzpunkt (°C) bzw. Brechungsindex ($n_D^{20}$) |
|---|---|---|---|---|---|
| 23 | Cl | Cl | $-CH - CH_2-$<br>$C_2H_5$ | | |
| 24 | Cl | H | $-CH - CH_2-$<br>$C_3H_7$-n | | |
| 25 | Cl | Cl | $-CH - CH_2-$<br>$C_3H_7$-n | | |
| 26 | Cl | H | $-CH - CH_2-$<br>$CH_2Cl$ | | |
| 27 | Cl | Cl | $-CH - CH_2-$<br>$CH_2Cl$ | | |
| 28 | Cl | H | $-CH - CH-$<br>$CH_3$ $CH_3$ | | |
| 29 | Cl | Cl | $-CH - CH-$<br>$CH_3$ $CH_3$ | | |
| 30 | Cl | H | $-CH_2-CH_2-CH_2-$ | | |
| 31 | Cl | Cl | $-CH_2-CH_2-CH_2-$ | | |
| 32 | Cl | Cl | $-CH_3$ | $-CH_3$ | 1,5481 |

Tabelle 1 (Fortsetzung)

| Bei-spiel Nr. | $X^1$ | $X^2$ | $R^1$ | $R^2$ | Schmelzpunkt (°C) Brechungsindex ($n_D^{20}$) |
|---|---|---|---|---|---|
| 33 | Cl | H | $-CH-CH_2-CH_2-CH_2-$<br>$CH_3$ | | |
| 34 | Cl | Cl | $-CH-CH_2-CH_2-CH_2-$<br>$CH_3$ | | |
| 35 | Cl | H | $-CH-CH_2-CH_2-CH_2-$<br>Cl | | |
| 36 | Cl | Cl | $-CH-CH_2-CH_2-CH_2-$<br>Cl | | |
| 37 | Cl | H | $-CH-CH_2-CH_2-CH-$<br>$CH_3$ $CH_3$ | | |
| 38 | Cl | Cl | $-CH-CH_2-CH_2-CH-$<br>$CH_3$ $CH_3$ | | |

In den nachfolgenden Verwendungsbeispielen wird die nachstehend angegebene Verbindung als Vergleichssubstanz eingesetzt:

(A) =

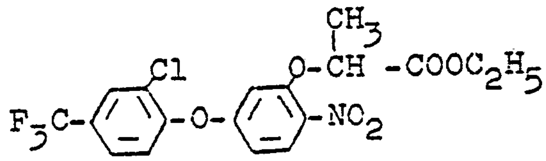

α-(5-(2-Chlor-4-trifluormethyl-phenoxy)-2-nitro-phenoxy)-propionsäure-ethylester
(bekannt aus DE-OS 2 311 638 bzw. US-PS 4 093 446)

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (1), (2) und (3) eine bessere selektive herbizide Wirksamkeit als die Vergleichsubstanz (A).

Beispiel B

Entlaubung und Austrocknung der Blätter bei Baumwolle

Lösungsmittel: 3o Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigen die erfindungsgemäßen Wirkstoffe (1) und (2) eine starke Wirkung.

0064658

## Patentansprüche

1. Substituierte Phenoxybenzaldehyd-acetale der Formel

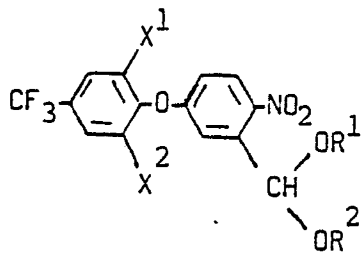

(I)

in welcher
$X^1$ für Wasserstoff oder Halogen steht,
$X^2$ für Wasserstoff oder Halogen steht,
$R^1$ für gegebenenfalls substituiertes Alkyl steht und
$R^2$ für gegebenenfalls substituertes Alkyl steht und außerdem
$R^1$ und $R^2$ gemeinsam für gegebenenfalls substituiertes Alkylen steht.

2. Substituierte Phenoxybenzaldehyd-acetale der Formel (I), in welcher $X^1$ für Chlor steht, $X^2$ für Wasserstoff oder Chlor steht, $R^1$ für gegebenenfalls ein- oder mehrfach durch Fluor, Chlor, Methoxy und/oder Ethoxy substituiertes geradkettiges oder verzweigtes Alkyl mit 1 bis 5 Kohlenstoffatomen steht und $R^2$ für gegebenenfalls ein-/oder mehrfach durch Fluor, Chlor, Methoxy und/oder Ethoxy substituiertes geradkettiges oder verzweigtes

Alkyl mit 1 bis 5 Kohlenstoffatomen steht und außerdem $R^1$ und $R^2$ gemeinsam für gegebenenfalls durch Alkyl mit 1 bis 3 Kohlenstoffatomen, Chloralkyl mit 1 oder 2 Kohlenstoffatomen und 1 bis 3 Chloratomen und/oder Chlor substituiertes Alkylen mit 2 bis 5 Kohlenstoffatomen stehen.

3. Verfahren zur Herstellung von substituierten Phenoxybenzaldehyd-acetalen der Formel

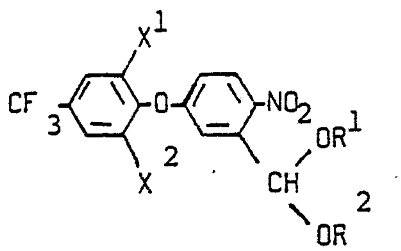

(I)

in welcher

$X^1$ für Wasserstoff oder Halogen steht,

$X^2$ für Wasserstoff oder Halogen steht,

$R^1$ für gegebenenfalls substituiertes Alkyl steht und

$R^2$ für gegebenenfalls substituiertes Alkyl steht und außerdem

$R^1$ und $R^2$ gemeinsam für gegebenenfalls substituiertes Alkylen stehen,

dadurch gekennzeichnet, daß man

(a) Phenoxybenzaldehyde der Formel

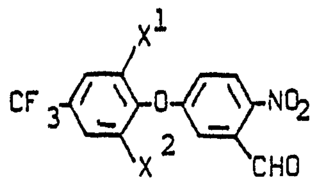

(II)

in welcher
$X^1$ und $X^2$ die oben angegebene Bedeutung haben,

entweder

α) mit Alkoholen der Formel

$R^1OH$ (IIIa)

in welcher
$R^1$ die oben angegebene Bedeutung hat, und/oder der Formel

$R^2OH$ (IIIb)

in welcher
$R^2$ die oben angegebene Bedeutung hat, bzw. mit Alkandiolen der Formel

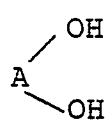

(IV)

in welcher
A für gegebenenfalls substituiertes Alkylen steht,

gegebenenfalls in Gegenwart eines Katalysators und gegebenenfalls in Gegenwart eines Verdünnungsmittels umsetzt,

oder

ß) mit Orthoameisensäureestern der Formel

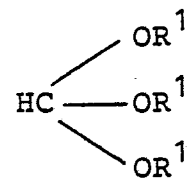

(V)

in welcher
$R^1$ die oben angegebene Bedeutung hat,

in Gegenwart eines Katalysators und gegebenenfalls in Gegenwart eines Verdünnungsmittels umsetzt,

oder

b) Phenoxybenzaldehyd-acetale der Formel

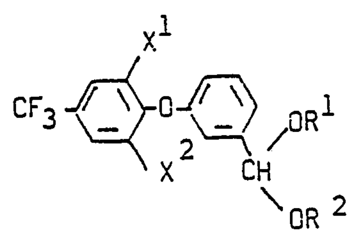

(VI)

in welcher
$X^1$, $X^2$, $R^1$ und $R^2$ die oben angegebene Bedeutung haben,

mit Salpetersäure gegebenenfalls in Gegenwart eines Katalysators und gegebenenfalls in Gegenwart eines Verdünnungsmittels umsetzt, oder

(c) Phenoxybenzaldehyd-acylale der Formel

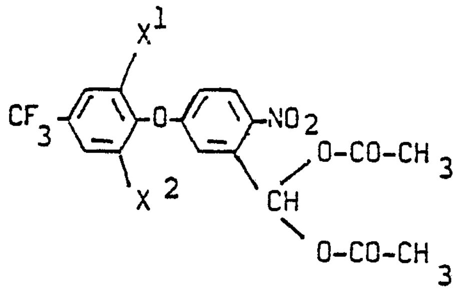

(VII)

in welcher
$X^1$ und $X^2$ die oben angegebene Bedeutung haben,

mit Alkoholen oder Formel

$R^1OH$ (IIIa)

in welcher
$R^1$ die oben angegebene Bedeutung hat,

und/oder der Formel

$R^2OH$ (IIIb)

in welcher
$R^2$ die oben angegebene Bedeutung hat,

bzw. mit Alkandiolen der Formel

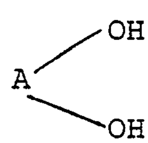

(IV)

in welcher
A die oben angegebene Bedeutung hat,

gegebenenfalls in Gegenwart eines Katalysators und gegebenenfalls in Gegenwart eines Verdünnungsmittels umsetzt, oder

(d) Phenole der Formel

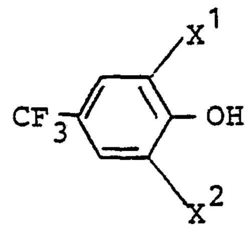

(VIII)

in welcher
$X^1$ und $X^2$ die oben angegebene Bedeutung haben,

mit 5-Halogen-2-nitro-benzaldehyd-acetalen der Formel

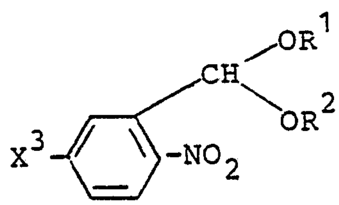

(IX)

in welcher
$R^1$ und $R^2$ die oben angegebene Bedeutung haben und
$X^3$ für Fluor, Chlor oder Brom steht,

gegebenenfalls in Gegenwart eines Säureakzeptors und gegebenenfalls in Gegenwart eines Verdünnungsmittels umsetzt.

4. Herbizide und pflanzenwuchsregulierende Mittel, gekennzeichnet durch einen Gehalt an mindestens einem substituierten Phenoxybenzaldehyd-acetal der Formel (I).

5. Verfahren zur Bekämpfung von Unkräutern sowie zur Regulierung des Pflanzenwachstums, dadurch gekennzeichnet, daß man substituierte Phenoxybenzaldehyd-acetale der Formel (I) auf Unkräuter bzw. die zu regulierenden Pflanzen und/oder deren Lebensraum ausbringt.

6. Verwendung von substituierten Phenoxybenzaldehyd-acetalen der Formel (I) zur Bekämpfung von Unkräutern sowie zur Regulierung des Pflanzenwachstums.

7. Verfahren zur Herstellung von herbiziden bzw. pflanzenwuchsregulierenden Mitteln, dadurch gekennzeichnet, daß man substituierte Phenoxybenzaldehyd-acetale der Formel (I) mit Streckmitteln und/oder oberflächenaktiven Stoffen vermischt.

8. Substituiertes Phenoxybenzaldehyd-acetal der Formel

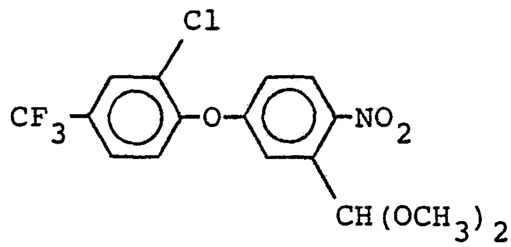

9. Substituiertes Phenoxybenzaldehyd-acetal der Formel

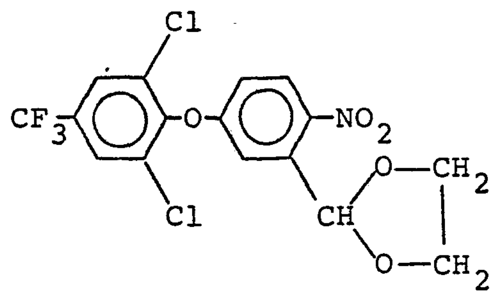

10. Substituiertes Phenoxybenzaldehyd-acetal der Formel

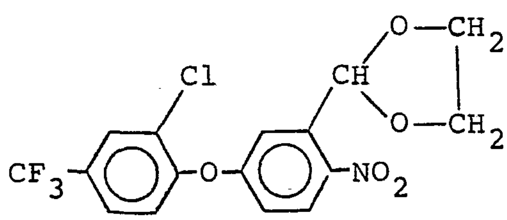

Europäisches Patentamt

# EUROPÄISCHER RECHERCHENBERICHT

0064658

Nummer der Anmeldung

EP 82 10 3514

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 023 725 (UBE INDUSTRIES)<br>* Ansprüche 1,21 * | 1,2,4-7 | C 07 C 79/35<br>C 07 D 317/22<br>C 07 C 76/02<br>A 01 N 35/04<br>A 01 N 43/24 //<br>C 07 D 319/06<br>C 07 D 321/06 |
| A | EP-A-0 021 868 (RHONE POULENC)<br>* Anspruch 1; Seite 5, Zeile 24 - Seite 13, Zeile 10 * | 1-3 | |
| A | GB-A-2 060 634 (CHEVRON RESEARCH)<br>* Ansprüche 1,6,8 * | 1-7 | |
| A | CHEMICAL ABSTRACTS, Band 91, Nr. 11, 10. September 1979, Seite 752, Nr. 91346k, Columbus, Ohio, USA<br>& JP - A - 79 32427 (MITSUI TOATSU CHEMICALS, INC.) 09-03-1979 * Zusammenfassung * | 1,2,4-7 | |

RECHERCHIERTE SACHGEBIETE (Int. Cl. 3)

C 07 C 79/00
C 07 D 317/00
C 07 D 319/00

Der vorliegende Recherchenbericht wurde für alle Patentansprüche erstellt.

| Recherchenort | Abschlußdatum der Recherche | Prüfer |
|---|---|---|
| DEN HAAG | 13-07-1982 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82